# EUROPEAN PATENT SPECIFICATION

(11) **EP 2 481 393 B1**
(45) Date of publication and mention of the grant of the patent: **02.11.2016**
(21) Application number: 10818676.8
(22) Date of filing: 06.09.2010
(51) Int. Cl.: A61K 8/88, A61K 8/06, A61K 8/29, A61K 8/35, A61K 8/41, A61K 8/49, A61K 8/81, A61Q 17/04, A61K 8/02

(54) **SUNSCREEN COSMETIC**
SONNENSCHUTZ-KOSMETIKUM
PRODUIT COSMÉTIQUE ÉCRAN SOLAIRE

(30) Priority: 24.09.2009 JP 2009218818
(43) Date of publication of application: 01.08.2012
(73) Proprietor: Shiseido Company, Ltd., Chuo-ku Tokyo 104-8010 (JP)
(72) Inventor: YAMAGUCHI Kazuhiro, Yokohama-shi Kanagawa 224-8558 (JP); ISHIDA Kahori, Yokohama-shi Kanagawa 224-8558 (JP)
(74) Representative: Ter Meer Steinmeister & Partner
(86) International application number: PCT/JP2010/065199
(87) International publication number: WO 2011/037000

(56) References cited:
- EP-A1- 2 027 847
- JP-A- 8 053 568
- JP-A- 9 208 437
- JP-A- 9 208 437
- JP-A- 11 049 655
- JP-A- 2004 124 045
- JP-A- 2007 145 722
- JP-A- 2007 182 388
- JP-A- 2007 182 389
- JP-A- 2007 277 167
- JP-A- 2007 320 917
- JP-A- 2008 208 052
- DATABASE WPI Week 199618 Thomson Scientific, London, GB; AN 1996-175774 XP002739433, & JP H08 53568 A (SEKISUI PLASTICS CO LTD) 27 February 1996 (1996-02-27)
- DATABASE WPI Week 199510 Thomson Scientific, London, GB; AN 1995-070204 XP002739434, & JP H06 345632 A (KAO CORP) 20 December 1994 (1994-12-20)

## Description

### TECHNICAL FIELD

The present invention relates to a sunscreen cosmetic. More specifically, it relates to a sunscreen cosmetic that prevents staining due to secondary adhesion to clothing.

### BACKGROUND ART

Important ultraviolet wavelength regions absorbed by sunscreen cosmetics are the UV-A region (320-400 nm) and UV-B region (290-320 nm). It was believed that the ultraviolet light in the UV-A region darkened the skin but it would not cause sunburn and accelerate aging of the skin as the ultraviolet light in the UV-B region would.

However, in recent years, it has been made clear that, whereas the ultraviolet light in the UV-B region only reaches the surface part of the skin, the ultraviolet light in the UV-A region reaches the deeper part of the skin and induces not only skin aging but also skin cancer. Therefore, there is an increasing demand for sunscreen cosmetics to have ultraviolet absorption in the UV-A region.

Examples of ultraviolet absorbents useful in the UV-A region (called UVA absorbents) include hexyl diethylaminohydroxybenzoylbenzoate, 2,4-bis{[4-(2-ethylhexyloxy)-2-hydroxy]-phenyl}-6-(4-methoxyphenyl)-1,3,5-triazine, 4-tert-butyl-4-methoxydibenzoylmethane, and 2-hydroxy-4-methoxybenzophenone.

One of the important issues for sunscreen cosmetics is the problem of staining (i.e. dyeing) from secondary adhesion of the ultraviolet absorbent having a color onto clothing; useful UVA absorbents such as hexyl diethylaminohydroxybenzoylbenzoate often stain due to the secondary adhesion.

For hexyl diethylaminohydroxybenzoylbenzoate, technology has been developed to prevent staining by blending in a combination of specific benzotriazole derivatives into the sunscreen cosmetic (Patent Document 1).

Also, octylmethoxy cinnamate is known as a useful ultraviolet absorbent for sunscreen cosmetics, but octylmethoxy cinnamate easily develops color and causes staining. For this, a technology has been developed in which octylmethoxy cinnamate and a specific benzotriazole derivative are used in combination to prevent staining (coloring) from octylmethoxy cinnamate and also manifest a superior ultraviolet absorption effect in the UV-A region as well (Patent Document 2).

As described thus far, sunscreen cosmetics containing ultraviolet absorbents are expected to solve the problem of staining.

Sunscreen cosmetics composed of a water-in-oil emulsified composition containing a UVA absorbent such as hexyl diethylaminohydroxybenzoylbenzoate is a prior art (Patent Documents 3 and 4); a sunscreen cosmetic often contains fine particles of zinc oxide and/or titanium oxide, as an ultraviolet scattering agent, in addition to the ultraviolet absorbent so as to increase the ultraviolet protection effect.

On the other hand, cosmetics that contain titanium oxide resin powder, i.e. resin containing inside titanium oxide powder, are widely known as makeup cosmetics. Relating to cosmetics to which ultraviolet blocking properties are given, for the purpose of using resin powder containing metal oxide particles to resolve the technical difficulty accompanying the use of metal oxide fine particles as a raw material of a cosmetic and the downside of using only organic ultraviolet absorbents, a practice is known that blends resin powder that contains inside metal oxide with ultraviolet blocking ability having an average particle size of 0.003-0.1 *µ*m into a cosmetic (Patent Document 5). Furthermore, relating to spherical resin suitable for visual light transmissive raw material and a preparation method thereof, as well as cosmetics that make use thereof, a practice is known that, by using resin powder containing metal compound particles, makes organic ultraviolet absorbents unnecessary and resolves the technical difficulty accompanying the use of metal oxide fine particles as a raw material of a cosmetic and the downside of the presence of organic ultraviolet absorbents (Patent Document 6).

### {Prior art documents}

### {Patent Documents}

| | |
|---|---|
| Patent Citation 1: | JP 2007-182388 A |
| Patent Citation 2: | JP 2005-206473 A |
| Patent Citation 3: | JP 2003-113020 A |
| Patent Citation 4: | JP 2007-530637 A |
| Patent Citation 5: | JP H09-208437 A |
| Patent Citation 6: | JP H08-53568 A |

### SUMMARY OF INVENTION

### PROBLEM THAT INVENTION IS TO SOLVE

The inventors conducted earnest research from the point of view of preventing staining from sunscreen cosmetic and discovered that blending a specific UVA absorbent selected from one, two or more of hexyl diethylaminohydroxybenzoylbenzoate, 2,4-bis{[4-(2-ethylhexyloxy)-2-hydroxy]-phenyl}-6-(4-methoxyphenyl)-1,3,5-triazine, 4-tert-butyl-4-methoxydibenzoylmethane, and 2-hydroxy-4-methoxybenzophenone, in combination with ultrafine particles of titanium oxide, into a sunscreen cosmetic could achieve an excellent ultraviolet protection effect, but staining from the secondary adhesion on clothing was difficult to remove with washing.

The inventors also discovered that this problem can be excellently solved by blending in said specific UVA absorbent in combination with resin spherical powder containing inside 35% or more of hydrophobicized ultrafine particles of titanium oxide, thus completing the present invention.

The object of the present invention is to provide a sunscreen cosmetic comprising a combination of an UVA absorbent and fine particle titanium oxide wherein the staining of clothing (dyeing) is improved.

### TECHNICAL SOLUTION

That is, the present invention provides a sunscreen cosmetic characteristically comprising:
(1) an UVA absorbent selected from one, two or more from hexyl diethylaminohydroxybenzoylbenzoate, 2,4-bis{[4-(2-ethylhexyloxy)-2-hydroxy]-phenyl}-6-(4-methoxyphenyl)-1,3,5-triazine, 4-tert-butyl-4-methoxydibenzoylmethane, and 2-hydroxy-4-methoxybenzophenone, and
(2) resin spherical powder containing inside 35% or more of hydrophobicized ultrafine particles of titanium oxide.

Also, the present invention provides the aforementioned sunscreen cosmetic wherein said hydrophobicized ultrafine particles of titanium oxide are treated with an aluminum salt of a fatty acid.

Furthermore, the present invention provides the aforementioned sunscreen cosmetic wherein said sunscreen cosmetic is a water-in-oil emulsified cosmetic.

### ADVANTAGEOUS EFFECTS

(1) The sunscreen cosmetic of the present invention reduces the staining of clothing. Therefore, an UVA absorbent and ultrafine particles of titanium oxide can be added to a sunscreen cosmetic at a high blend ratio.
(2) The sunscreen cosmetic of the present invention manifests a superior ultraviolet protection effect because of the UVA absorbent and the ultrafine particles of titanium oxide.

### BRIEF DESCRIPTION OF DRAWINGS

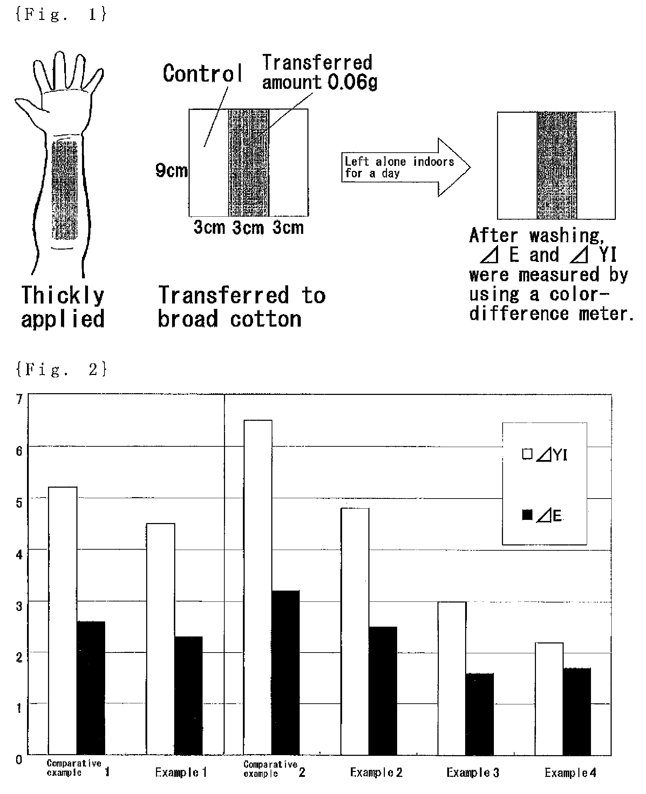

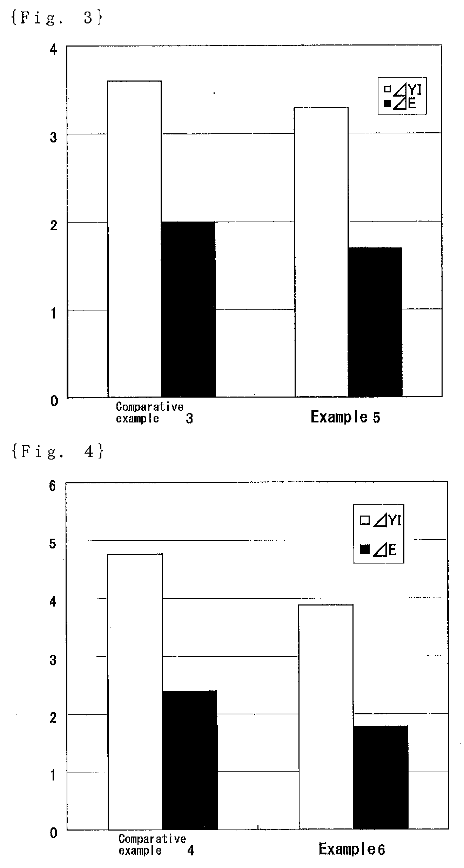

{FIG. 1} FIG. 1 illustrates how to measure the stain.
{FIG. 2} FIG. 2t shows the measurement results of the stain of Table 1.
{FIG. 3} FIG. 3 shows the measurement results of the stain of Table 2.
{FIG. 4} FIG. 4 shows the measurement results of the stain of Table 3.

### MODE FOR CARRYING OUT THE INVENTION

The present invention is described in detail below.

### "Hexyl diethylaminohydroxybenzoylbenzoate"

Hexyl diethylaminohydroxybenzoylbenzoate used in the present invention is a prior art ultraviolet absorbent in the UV-A region (UVA absorbent). In the present invention, a commercial product (Uvinul A plus from BASF) can be preferably used.

### "2,4-bis{[4-(2-ethylhexyloxy)-2-hydroxy]-phenyl}-6-(4-methoxyphenyl)-1,3,5-triazine"

2,4-bis{[4-(2-ethylhexyloxy)-2-hydroxy]-phenyl}-6-(4-methoxyphenyl)-1,3,5-triazine used in the present invention is a prior art ultraviolet absorbent in the UV-A region (UVA absorbent). In the present invention, a commercial product (Tinosorb S from Ciba Specialty Chemicals Co., Ltd.) can be preferably used.

### "4-tert-butyl-4-methoxydibenzoylmethane"

4-tert-butyl-4-methoxydibenzoylmethane used in the present invention is a prior art ultraviolet absorbent in the UV-A region (UVA absorbent). In the present invention, a commercial product (Parsol from DSM) can be preferably used.

### "2-hydroxy-4-methoxybenzophenone"

2-hydroxy-4-methoxybenzophenone used in the present invention is a prior art ultraviolet absorbent in the UV-A region. In the present invention, a commercial product (Uvinul M40 from BASF) can be preferably used.

The blend ratios of the aforementioned UVA absorbents are determined as appropriate; the total blend ratio of one, two, three, or four of them is usually 1-35 wt% relative to the total amount of the sunscreen cosmetic. A preferable range is 3-30 wt% and more preferable is 5-20 wt%.

In addition to the aforementioned UVA absorbents, it is preferable to also blend octylmethoxy cinnamate as a UVB absorbent into the sunscreen cosmetic of the present invention. The blend ratio is determined as appropriate; it is usually, relative to the total amount of the present invention, 1-40 wt%, and preferably 5-20 wt%.

### "Resin spherical powder"

The resin spherical powder used in the present invention is a resin spherical powder containing inside 35% or more of hydrophobicized ultra fine particles of titanium oxide.

The ultrafine particles of titanium oxide in the present invention are ultrafine particle titanium oxide powder having an average particle size of 10-100 nm. The average particle size value is measured with a conventional method using a laser particle size distribution meter.

Resin of the resin spherical powder is resin powder that is spherical resin containing inside 41 wt% or more of hydrophobicized ultrafine particles of titanium oxide. The resin powder that is spherical resin powder containing inside ultrafine particles of titanium oxide a prior art; however, if the content is 34 wt% or less, then the content is too low; for example, even if one tried to give ultraviolet protection ability to a cosmetic by blending resin powder containing ultrafine particles of titanium oxide into the cosmetic, one would have to blend in a large amount of the resin powder to achieve sufficient performance, which would make the blend design of the cosmetic quite difficult.

Also, if non-hydrophobicized ultrafine particles of titanium oxide are used, when preparing the dispersion liquid before polymerization, the viscosity of this dispersion liquid becomes very high and the fine particles of the metal compound cannot get dispersion energy effectively and it becomes difficult to turn the metal compound fine particles into a highly dispersed state. Therefore, homogeneous resin powder cannot be prepared because metal compound aggregates remain and/or the powder becomes inhomogeneous.

Powder that contains inside 41 wt% or more of ultrafine particles of titanium oxide has never been used as a raw material for cosmetics.

### <Preparation method of resin spherical powder>

Known methods to prepare resin spherical powder include the suspension polymerization method, the emulsification polymerization method, a method in which a resin solution is mechanically dispersed or emulsified, and a method in which fine particles are precipitated from a resin solution. An example of a method to disperse hydrophobicized fine particles of titanium oxide inside the mother material particles to a content over 35 wt%, is a method, when the mother material particles are nylon resin, in which cyclic lactam is heated and dissolved in paraffin and such, to which a desired amount of fine powder of titanium dioxide is added, and, as stirring is being done, a polymerization accelerator such as phosphorus trichloride is added to do alkaline polymerization, and the obtained particles are separated by means of filtration and washed with an organic solvent such as benzene and isopropyl alcohol, followed by drying.

An example of a method when the mother material particles are silicone resin is a method in which fine particles of titanium dioxide or zinc oxide are added to and mixed in an aqueous solution of ammonia, amine and such, and a hydrolysable silane, such as chlorosilane, hydrogen silane, alkoxysilane, and acetoxysilane is added for the hydrolysis reaction and condensation reaction, and the obtained particles are separated by means of filtration, washed with water, and dried.

An example of a method when the mother material particles are silicon oxide is a method in which fine powder of titanium dioxide and/or zinc oxide is added to sodium silicate to prepare a suspension liquid, a mixed liquid of a surfactant and an oil based dispersing agent such as benzene is prepared, the aforementioned suspension liquid is added to this mixed liquid to emulsify it to obtain a water-in-oil emulsion, which is reacted with sodium silicate by adding a salt such as ammonium sulfate and ammonium chloride, and the obtained particles are separated by means of filtration, washed with water, rinsed with an organic solvent such as methanol, and dried.

Furthermore, an example of a preferable method is a method in which non-water soluble thermoplastic resin is turned into a liquid state at a high temperature around 200° C, into which hydrophobicized fine particles of titanium oxide are dispersed, said dispersion liquid is dispersed into a water soluble material (such as an oligosaccharide) and cooled and solidified, and the water soluble materials are removed by means of washing with water to obtain resin spherical powder composed of non-water soluble thermoplastic resin and hydrophobicized fine particles of titanium oxide.

The average particle size of the resin spherical powder is 1-10 *µ*m, preferably 1-5 *µ*m. The average particle size value is measured with a conventional method using a laser particle size distribution meter.

Selection of the treatment agent for the hydrophobicizing treatment is not limited; preferable examples include aluminum stearate, methyl hydrogen polysiloxane, and alkyl triethoxysilane; the hydrophobicizing treatment is done with a conventional method.

The blend ratio of the resin spherical powder is usually 1-40 wt% relative to the total amount of the sunscreen cosmetic. A preferable range is 3-30 wt% and more preferable is 5-20 wt%.

### "Preparation of a sunscreen cosmetic as a water-in-oil or oil-in-water emulsified composition"

In addition to the aforementioned essential ingredients, other ingredients commonly used in cosmetics can be blended in as necessary in the sunscreen cosmetic of the present invention; preferably, such ingredients, examples of which include whitening agents, humectants, antioxidants, alcohols, thickeners, coloring agents, various powder ingredients, various oil-based ingredients, and various water-based ingredients, are blended in as necessary and an emulsified composition, preferably a water-in-oil emulsified composition, should preferably be prepared with a conventional method.

The blend ratio of water in the water-in-oil emulsified composition or the oil-in-water emulsified composition is not limited and determined appropriately for the product.

Any emulsifier can be used. Examples of the emulsifier include POE/methylpolysiloxane copolymer, silicone chain branched POE/methylpolysiloxane, cross-linked POE/mothylpolysiloxane copolymer, alkyl/POE co-modified methylpolysiloxane copolymer, silicone chain branched alkyl/POE comodified methylpolysiloxane copolymer, glycerin fatty acid ester, polyglycerin fatty acid ester, polyoxyethylene glycerin fatty ester, sorbitan fatty acid ester, and polyoxyethylene sorbitan fatty acid.

Of these, a preferable emulsifier for the present invention is alkyl/POE comodified methylpolysiloxane or acrylic acid/alkyl methacrylate copolymer.

Specific examples of the ingredients that can be blended in within the range that does not adversely affect the effect of the present invention are shown below:
Oil components such as decamethylcyclopentasiloxane, dimethylpolysiloxane, heptamethyloctyltrisiloxane, trimethylsiloxysilicic acid, liquid paraffin, squalane, avocado oil, macadamia nut oil, corn oil, olive oil, rapeseed oil, evening primrose oil, castor oil, sunflower oil, tea seed oil, rice bran oil, jojoba oil, cacao oil, coconut oil, squalene, beef tallow, Japanese core wax, beeswax, candelilla wax, carnauba wax, whale wax, lanolin, liquid paraffin, polyoxyethylene (8 mole) oleyl alcohol ether, glyceryl monooleate, cyclomethicone, dimethylpolysiloxane, and diphenylpolysiloxane.

Higher alcohols such as caprylic alcohol, lauryl alcohol, myristyl alcohol, cetyl alcohol, cholesterol, and phytosterol.

Higher fatty acids such as caprylic acid, lauric acid, myristic acid, palmitic acid, stearic acid, behenic acid, lanolin fatty acid, linoleic acid, and linolenic acid.

Humectants such as polyethylene glycol, glycerin, sorbitol, xylitol, maltitol, mucopolysaccharide, hyaluronic acid, chondroitin sulfate, and chitosan.

Thickeners such as methyl cellulose, ethyl cellulose, Arabic gum, and polyvinyl alcohol.

Liquid that constitutes the water phase such as ethanol and 1,3-butylene glycol.

Antioxidants such as butylhydroxytoluene, tocopherol, and phytic acid.

Antibacterial preservatives such as benzoic acid, salicylic acid, sorbic acid, paraoxybenzoic esters (ethylparaben and butylparaben, for example), and hexachlorophene.

Amino acids such as glycine, alanine, valine, leucine, serine, threonine, phenyalanine, tyrosine, aspartic acid, asparagine, glutamine, taurine, arginine, and histidine, as well as hydrochlorides thereof.

Organic acids such as acyl sarcosinic acid (sodium lauroyl sarcosinate, for example), glutathione, citric acid, malic acid, tartaric acid, and lactic acid.

Vitamins such as vitamin A and its derivatives, vitamin B's including vitamin B6 hydrochloride, vitamin B6 tripalmitate, vitamin B6 dioctanoate, vitamin B2 and its derivatives, vitamin B12, and vitamin B15 and its derivatives, vitamin C's including ascorbic acid, ascorbic malic esters (salts), and ascorbic dipalmitate, vitamin E's including *α*-tocopherol, *β*-tocopherol, γ-tocopherol, vitamin E acetate, and vitamin E nicotinate, vitamin D's, vitamin H, pantothenic acid, and pantethine.

Various drugs such as nicotinamide, benzyl nicotinate, γ-oryzanol, allantoin, glycyrrhizic acid (salt), glycyrrhizic acid and its derivatives, hinokitiol, musidine, bisabolol, eucalyptol, thymol, inositol, saponins (saikosaponin, carrot saponin, gourd saponin, soapberry saponin, etc.), pantothenylethyl ether, ethynylestradiol, tranexamic acid, cepharanthine, and placenta extract.

Natural extracts from Rumex japonicus, Sophora flavescens, Nuphar japonica, orange, sage, thyme, yarrow, mallow, smilax, swertia, Ligusticum acutilobum, bitter orange peel, birch, horsetail, gourd, horse chestnut, creeping saxifrage, arnica, lily, mugwort, Paeonia lactiflora, aloe, gardenia, Chamaecyparis pisifera, etc. extracted by using an organic solvent, alcohol, polyhydric alcohol, water, hydroalcohol, etc.

Cationic surfactants such as stearyltrimethylammonium chloride, benzalkonium chloride, and lauryl amine oxide.

Sequestering agents such as disodium edetate, trisodium edetate, sodium citrate, sodium polyphosphate, sodium metaphosphate, and gluconic acid.

The sunscreen cosmetic of the present invention can be used in any product form such as ointments, creams, emulsions, and lotions.

### EXAMPLES

The present invention is further described in detail below by referring to Examples. The present invention is not limited to these examples. The blend ratios are in relation to the total amount and in weight-percentage units unless specified otherwise.

The water-in-oil emulsified cosmetics (emulsion sunscreen) shown in the tables are prepared using a conventional method. The following method was used to confirm the rinse removal effect against staining. Table 3 is an oil-in-water emulsified cosmetic (emulsion sunscreen).

### <Preparation method of resin spherical powder> (Preparation Example 1)

25 weight parts of polyamide 12 and 25 weight parts of fine particles of titanium oxide treated with aluminum stearate (average particle size 15 nm, TT0 S4 from Ishihara Sangyo Kaisha Ltd.) were dispersed by means of an ultrasonic dispersing device, which were then heated up to 200° C and mixed with 100 weight parts of an oligosaccharide, which are then melted and kneaded with an extruder, and extruded in a strand form. This was transported and fed into a container having 450 weight parts of water to dissolve the oligosaccharide into water and obtain a water dispersion liquid of polyamide particles containing inside titanium oxide.

The obtained water dispersion liquid was then filtered with 5C paper filter. The recovered filtrate was dispersed again in water such that the content was 5 wt% and filtration was done again. This operation was repeated three times for rinsing, followed by drying, to obtain polyamide particles containing inside titanium oxide (average particle size 5 *µ*m).

### (Preparation Example 2)

### Resin monomer phase

Fine particles of titanium oxide treated with aluminum stearate (Average particle size 15 nm MT-100T made by Tayca) 45 weight parts Vinyl acetate (resin monomer) 10 weight parts Methyl methacrylate (resin monomer) 34.8 weight parts Ethylene glycol dimethacrylate (resin monomer) 10 weight parts 2,2'-azobis 2,4-dimethylvaleronitrile (polymerization starter) 0.2 weight parts Water phase Water 500 weight parts Polyvinyl alcohol (degree of saponification 87%) 10 weight parts

The resin monomer phase was dispersed by using an ultrasonic dispersion device, which was added to the water phase and stirred with a homomixer to adjust the monomer particle size to be 4 *µ*m. This dispersion was then transferred to a reaction apparatus equipped with a stirrer and a thermometer; the temperature was raised to 55° C to start the polymerization. The polymerization was continued for five hours at this temperature, and the temperature was cooled down to room temperature; the obtained resin powder was separated by means of suction filtration. After rinsing with an appropriate amount of warm water and methanol, it was dried at room temperature to obtain resin spherical powder containing inside 45% of titanium oxide treated with aluminum stearate.

### "Method for measuring the staining (dyeing) tendency"

As shown in FIG. 1, the sample was applied thickly on an arm and then transferred to the middle of broad cotton (transferred amount approximately 0.06 g); after being left alone indoors for a day, it was washed using a conventional laundry detergent (Attack from Kao corporation) and 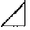 E and 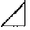 YI were measured by using a spectrophotometer (CM-2002 from Minolta, currently Konika Minolta Sensing Co., Ltd.). The results are shown in the tables and the figures.

In the following Examples, the staining tendency is reduced by replacing hydrophobicized fine particles of titanium oxide with resin containing inside titanium oxide and superior anti-staining properties are manifested.

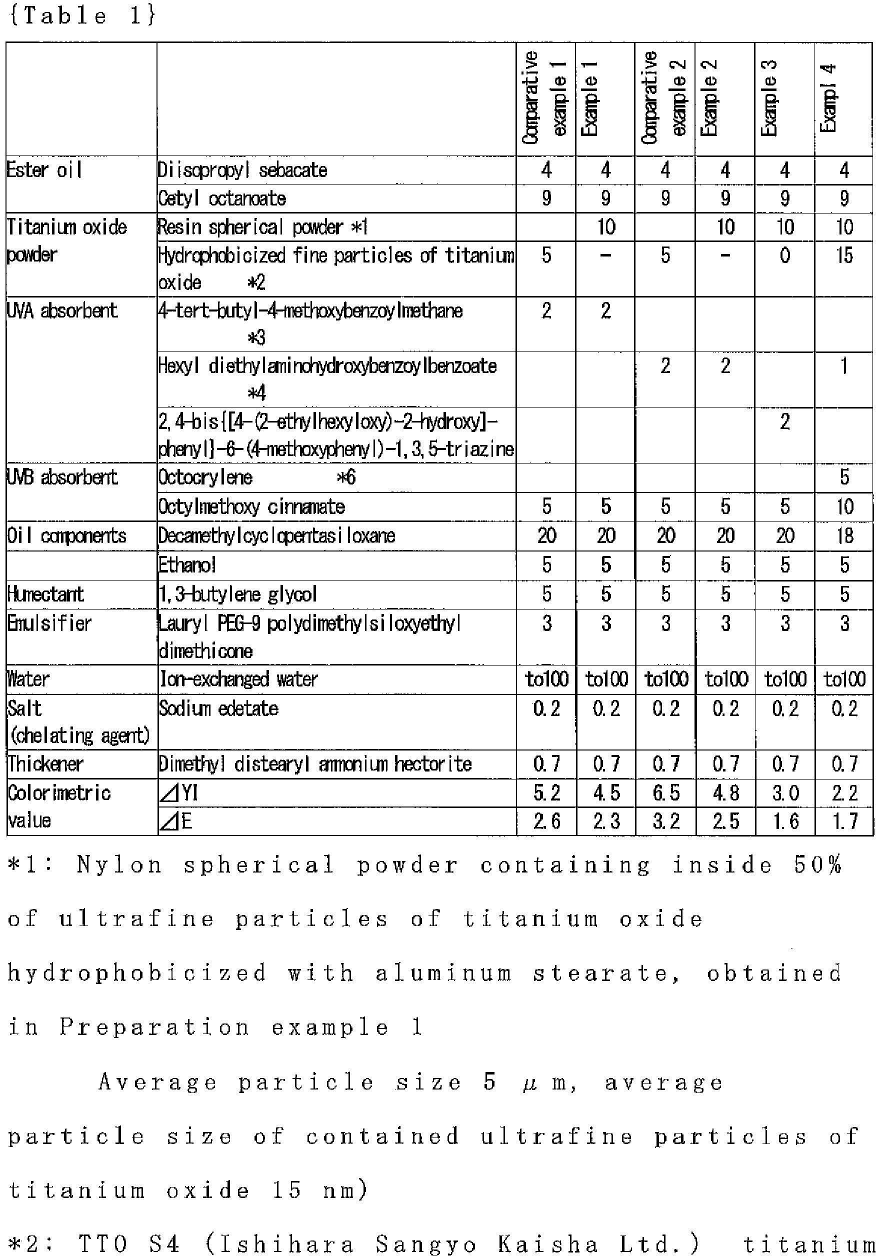

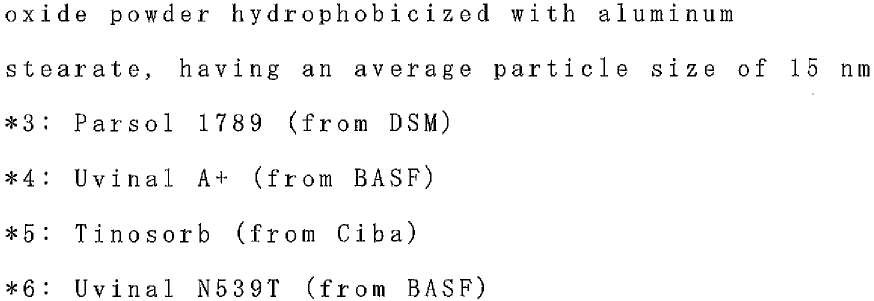

**{Table 2}**

| | | Comparative example 3 | Example 5 |
|---|---|---|---|
| Ester oil | Diethylhexyl succinate | 4 | 4 |
| | Trimethylhexanoin | 8 | 8 |
| | Octyl palmitate | 1 | 1 |
| Titanium oxide powder | Resin spherical powder *1 | | 4 |
| | Hydrophobicized fine particles of titanium oxide *2 | 1.8 | - |
| UVA absorbent | 2-hydroxy-4-methoxybenzophenone *3 | 3 | 3 |
| UVB absorbent | Octylmethoxy cinnamate | 10 | 10 |
| Oil components | Isohexadecane | 5 | 5 |
| | Decamethylcyclopentasiloxane | 9.1 | 9.1 |
| | Ethanol | 5 | 5 |
| Hunectant | 1,3-butylene glycol | 5 | 5 |
| Emulsifier | Lauryl PEG-9 polydimethylsiloxyethyl dimethicone | 2 | 2 |
| Water | Ion-exchanged water | to100 | to100 |
| Salt (chelating agent) | Edetic acid | 0.2 | 0.2 |
| Thickener | Dimethyl distearyl ammonium hectorite | 0.7 | 0.7 |
| Colorimetric value | 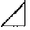 YI | 3.6 | 3.3 |
| | 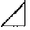 E | 2.0 | 1.7 |

| | | | |
|---|---|---|---|
| *1: Polyacrylic acid spherical powder containing inside 45% of ultrafine particles of titanium oxide hydrophobicized with aluminum stearate, obtained in Preparation example 2 | | | |

Average particle size 4 *µ*m, average particle size of contained ultrafine particles of titanium oxide 15 nm)
*2: MT-100T (from Tayca) Titanium oxide powder hydrophobicized with aluminum stearate, having an average particle size of 15 nm
*3: Uvinul M40 from BASF

In the aforementioned Examples, the staining tendency is reduced by replacing hydrophobicized fine particles of titanium oxide with the spherical resin powder of the present invention, and superior anti-staining properties are manifested.

### "Testing of the sunscreen cosmetic composed of an oil-in-water emulsified composition"

**{Table 3}**

| | Comparative example 4 | Example 6 |
|---|---|---|
| Ion-exchanged water | to100 | to100 |
| Dipropylene glycol | 3 | 3 |
| Dynamite glycerin | 1 | 1 |
| Polyoxyalkylene (C2-8) monoalkyl (C1-24) | 0.4 | 0.4 |
| Alkyl acrylate/methacrylate copolymer | 0.05 | 0.05 |
| Carboxyvinyl polymer | 0.1 | 0.1 |
| Potassium hydroxide | 0.055 | 0.055 |
| Hexyl diethylaminohydroxybenzoylbenzoate *1 | 2 | 2 |
| Octylmethoxy cinnamate | 5 | 5 |
| Isostearic acid | 0.2 | 0.2 |
| Titanium oxide MT-100T (from Tayca) *2 | 4.5 | |
| Resin spherical powder of the present invention *3 | | 10 |
| 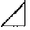 YI | 4.77 | 3.88 |
| 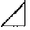 E | 2.40 | 1.78 |

| | | |
|---|---|---|
| *1: Uvinal A+ (from BASF) *2: MT-100T (from Tayca) Titanium oxide powder hydrophobicized with aluminum stearate, having an average particle size of 15 nm *3: Polyacrylic acid spherical powder containing inside 45% of ultrafine particles of titanium oxide hydrophobicized with aluminum stearate, obtained in Preparation example 2 | | |

In the aforementioned Examples, even when the sunscreen cosmetic is an oil-in-water emulsified composition, the staining tendency is reduced by replacing hydrophobicized fine particles of titanium oxide with the spherical resin powder of the present invention, and superior anti-staining properties are manifested.

### INDUSTRIAL APPLICABILITY

The present invention can provide a sunscreen cosmetic that has a high ultraviolet protection effect and prevents staining of clothing due to secondary adhesion.

## Claims

1. A sunscreen cosmetic comprising:
(1) an UVA absorbent selected from one, two or more from hexyl diethylaminohydroxybenzoylbenzoate, 2,4-bis{[4-(2-ethylhexyloxy)-2-hydroxy]-phenyl}-6-(4-methoxyphenyl)-1,3,5-triazine,4-tert-butyl-4-methoxydibenzoylmethane, and 2-hydroxy-4-methoxybenzophenone, and
(2) resin spherical powder containing inside 35% or more of hydrophobicized ultra fine particles of titanium oxide, wherein the ultra fine particles of titanium oxide have an average particle size of 10-100 nm.

2. The sunscreen cosmetic of claim 1, further comprising an UVB absorbent of octylmethoxy cinnamate in an amount of 5-20 wt.%.

3. The sunscreen cosmetic of claim 1 or 2, wherein said hydrophobicized ultrafine particle titanium oxide is treated with an aluminum salt of a fatty acid.

4. The sunscreen cosmetic of any one of claims 1 to 3, wherein said sunscreen cosmetic is a water-in-oil emulsified cosmetic.

## Patentansprüche

1. Sonnenschutzkosmetikum, umfassend:
(1) ein UVA-Absorptionsmittel, gewählt aus einem, zwei oder mehreren aus Hexyldiethylaminohydroxybenzoylbenzonat, 2,4-Bis{[4-(2-ethylhexyloxy)-2-hydroxy]-phenyl}-6-(4-methoxyphenyl)-1,3,5-triazin, 4-tert-Butyl-4-methoxydibenzoylmethan und 2-Hydroxy-4-methoxybenzophenon, und
(2) kugelförmiges Harzpulver, enthaltend im Inneren 35 % oder mehr hydrophobierte, ultrafeine Teilchen aus Titanoxid, wobei die ultrafeinen Teilchen aus Titanoxid eine durchschnittliche Teilchengröße von 10 - 100 nm besitzen.

2. Sonnenschutzkosmetikum nach Anspruch 1, weiterhin umfassend ein UVB-Absorptionsmittel aus Octylmethoxycinnamat in einer Menge von 5 - 20 Gew.-%.

3. Sonnenschutzkosmetikum nach Anspruch 1 oder 2, wobei das hydrophobierte, ultrafeine Titanoxidteilchen mit einem Aluminiumsalz einer Fettsäure behandelt ist.

4. Sonnenschutzkosmetikum nach mindestens einem der Ansprüche 1 - 3, wobei das Sonnenschutzkosmetikum ein Wasser-in-Öl-emulgiertes Kosmetikum ist.

## Revendications

1. Un produit cosmétique d'écran solaire comprenant:
(1) un agent absorbant les UVA choisi parmi un, deux ou plus de: l'hexyldiéthylaminohydroxybenzoylbenzoate, la 2,4-bis{[4-(2-éthylhexyloxy)-2-hydroxy]-phényl}-6-(4-méthoxyphényl)-1,3,5-triazine, le 4-tert-butyl-4-méthoxydibenzoylméthane et le 2-hydroxy-4-méthoxybenzophénone, et
(2) une poudre sphérique de résine contenant à l'intérieur de 35% ou plus de particules ultrafines rendues hydrophobes d'oxyde de titane, dans lequel les particules ultrafines d'oxyde de titane ont une taille de particules moyenne de 10 - 100 nm.

2. Le produit cosmétique d'écran solaire selon la revendication 1, en outre comprenant un agent absorbant les UVB d'octylméthoxycinnamate en une quantité de 5 - 20% en poids.

3. Le produit cosmétique d'écran solaire selon la revendication 1 ou 2, dans lequel ledit oxyde de titane de particules ultrafines rendu hydrophobe est traité par un sel d'aluminium d'un acide gras.

4. Le produit cosmétique d'écran solaire selon l'une quelconque des revendications 1 à 3, dans lequel ledit produit cosmétique d'écran solaire est un produit cosmétique en émulsion eau-dans-huile.
